# EUROPÄISCHE PATENTSCHRIFT

(11) **EP 3 079 736 B1**
(45) Veröffentlichungstag und Bekanntmachung des Hinweises auf die Patenterteilung: **26.09.2018**
(21) Anmeldenummer: 14809618.3
(22) Anmeldetag: 02.12.2014
(51) Int. Cl.: A61M 1/16, A61M 1/34

(54) **VORRICHTUNG ZUR EXTRAKORPORALEN BLUTBEHANDLUNG**
APPARATUS FOR EXTRACORPOREAL BLOOD TREATMENT
DISPOSITIF DE TRAITEMENT EXTRACORPOREL DU SANG

(30) Priorität: 13.12.2013 DE 102013021012
(43) Veröffentlichungstag der Anmeldung: 19.10.2016
(73) Patentinhaber: Fresenius Medical Care Deutschland GmbH, 61352 Bad Homburg (DE)
(72) Erfinder: WIKTOR, Christoph, 63571 Gelnhausen (DE); PETERS, Arne, Bad Homburg 61352 (DE)
(74) Vertreter: Oppermann, Frank
(86) Internationale Anmeldenummer: PCT/EP2014/076300
(87) Internationale Veröffentlichungsnummer: WO 2015/086382

(56) Entgegenhaltungen:
- EP-A1- 1 867 353
- WO-A1-2011/157396
- US-A- 5 011 607
- US-A- 5 863 421
- US-A1- 2011 132 838
- TORU SHINZATO ET AL: "Alternate repetition of short fore- and backfiltrations reduces convective albumin loss", KIDNEY INTERNATIONAL,, vol. 50, no. 2, 1 August 1996 (1996-08-01) , pages 432-435, XP001599012, DOI: HTTP://DX.DOI.ORG/10.1038/KI.1996.333

## Beschreibung

Die Erfindung betrifft eine Vorrichtung zur extrakorporalen Blutbehandlung.

Die bekannten Blutbehandlungsvorrichtungen verfügen über einen Dialysator oder Filter, der durch eine semipermeable Membran in eine Blutkammer und eine Dialysierflüssigkeitskammer getrennt ist. Das Blut des Patienten strömt in einem extrakorporalen Blutkreislauf durch die Blutkammer des Dialysators, während die Dialysierflüssigkeit in einem Dialysierflüssigkeitskreislauf durch die Dialysierflüssigkeitskammer des Dialysators strömt.

Während der extrakorporalen Blutbehandlung kann dem Patienten mit den bekannten Blutbehandlungsvorrichtungen über die semipermeable Membran des Dialysators Flüssigkeit entzogen oder Flüssigkeit zugeführt werden. Dem Patienten wird Flüssigkeit entzogen (Ultrafiltration), wenn der Druck auf der Blutseite der semipermeablen Membran größer als der Druck auf der Dialysierflüssigkeitsseite der Membran ist. Wenn der Druck auf der Blutseite hingegen kleiner als auf der Dialysierflüssigkeitsseite ist, wird dem Patienten Flüssigkeit zugeführt.

Die US 5 011 607 A1 beschreibt eine besondere Variante der extrakorporalen Blutbehandlung, bei der die Blutbehandlungsvorrichtung mit einem oszillierenden Transmembrandruck betrieben wird, so dass über die semipermeable Membran des Dialysators wechselweise Flüssigkeit dem extrakorporalen Blutkreislauf entzogen bzw. dem extrakorporalen Blutkreislauf zugeführt wird. Dadurch wird erreicht, dass sich Ablagerungen von der Membran lösen können.

Die aus der US 5 011 607 A1 bekannte Dialysevorrichtung weist eine zu der Dialysierflüssigkeitskammer des Dialysators führende Zuflussleitung und eine von der Dialysierflüssigkeitskammer abgehende Abflussleitung auf, wobei in der Zuflussleitung eine erste Dialysierflüssigkeitspumpe zum Fördern von Dialysierflüssigkeit in die Dialysierflüssigkeitskammer und in der Abflussleitung eine zweite Dialysierflüssigkeitspumpe zum Fördern von Dialysierflüssigkeit aus der Dialysierflüssigkeitskammer angeordnet sind. Wenn die Förderraten beider Dialysierflüssigkeitspumpen gleich sind, wird dem Patienten keine Flüssigkeit entzogen bzw. zugeführt. Die Dialysevorrichtung verfügt über eine Verdrängerpumpe, die eine Pumpenkammer aufweist, in der ein Verdrängerkörper angeordnet ist. Die Pumpenkammer der Kolbenpumpe steht mit der Zuflussleitung in Strömungsverbindung. Wenn die Kolbenpumpe läuft, wird ein oszillierender Transmembrandruck erzeugt, so dass in kurzen Zeitintervallen, d.h. in der Saugphase bzw. Druckphase der Pumpe, eine bestimmte Menge an Flüssigkeit dem extrakorporalen Blutkreislauf entzogen bzw. zugeführt wird. Diese Variante der Dialyse wird auch als push-pull Dialyse bezeichnet.

Die US 5 011 607 A1 beschreibt auch eine alternative Ausführungsform, bei der ein oszillierender Transmembrandruck nicht mit einer zusätzlichen push-pull Pumpe erzeugt wird. Bei der alternativen Ausführungsform werden die beiden Dialysierflüssigkeitspumpen zur Erzeugung des oszillierenden Transmembrandrucks wechselweise mit unterschiedlichen Förderraten betrieben.

Um dem Patienten über die gesamte Behandlungsdauer der extrakorporalen Blutbehandlung Flüssigkeit entziehen zu können, verfügen die bekannten Blutbehandlungsvorrichtungen über eine Ultrafiltrationseinrichtung, die eine Ultrafiltrationspumpe aufweist, mit der Flüssigkeit (Ultrafiltrat) aus dem Dialysierflüssigkeitssystem abgezogen wird.

Eine Blutbehandlungsvorrichtung mit einer Ultrafiltrationseinrichtung, die einen Betrieb im push-pull-Modus vorsieht, ist aus Replacement of renal function by dialysis, 5th ed./edited by Walter H. Hörl... [et al.], ISBN 1-4020-0083-9, S. 388,389 (Fig. 42) bekannt. Die bekannte Dialysevorrichtung verfügt sowohl über eine Ultrafiltrat-Pumpe als auch eine push-pull-Pumpe, wobei die Ultrafiltrat-Pumpe mit der Dialysierflüssigkeitsabflussleitung und die push-pull Pumpe mit der Dialysierflüssigkeits-Zuflussleitung in Fluidverbindung steht.

Die US 2011/132838 A1 beschreibt eine Blutbehandlungsvorrichtung mit einer Ultrafiltrationseinrichtung, die eine Ultrafiltrationspumpe aufweist. In der Dialysierflüssigkeitszuführleitung befindet sich eine erste Dialysatpumpe und in der Dialysierflüssigkeitsabführleitung befindet sich eine zweite Dialysatpumpe. Eine dritte Pumpe befindet sich in einer von der Dialysierflüssigkeitsabführleitung stromauf der zweiten Dialysatpumpe abzweigenden Leitung, die zu einem Ablauf führt. In der US 2011/132838 A1 wird vorgeschlagen, die dritte Pumpe wechselweise in beide Richtungen zu betreiben, so dass dem Patienten Flüssigkeit entzogen oder zugeführt werden kann.

Die EP 1 867 353 A1 beschreibt eine Butbehandlungsvorrichtung, die eine erste Pumpe in der Dialysierflüssigkeitszuführleitung und eine zweite Pumpe in der Dialysierflüssigkeitsabführleitung vorsieht, wobei im Dialysierflüssigkeitssystem eine Bypassleitung vorgesehen ist, in der sich eine dritte Pumpe befindet. Die Bypassleitung verbindet den zu der zweiten Pumpe führenden Abschnitt der Dialysierflüssigkeitsabführleitung mit dem von der zweiten Pumpe abgehenden Abschnitt der Dialysierflüssigkeitsabführleitung. Die dritte Pumpe wird für eine Rückfiltration verwendet, wenn sich der Filter zugesetzt hat, so dass der Transmembrandruck TMP über einen Grenzwert angestiegen ist. Zum Spülen des Filters wird die Förderrichtung der dritten Pumpe umgekehrt. Nach der Rückfiltration wird wieder in den Normalbetrieb umgeschaltet.

Die US 5 863 421 A beschreibt eine Blutbehandlungsvorrichtung, die über eine Ultrafiltrat-Pumpe verfügt, die dem Patienten entzogene Flüssigkeit in einen Ultrafiltrat-Tank pumpt. Die Blutbehandlungsvorrichtung sieht eine Unterbrechung der Blutbehandlung für die Durchführung einer Rückfiltration vor. Für diesen Betriebsmodus wird die Förderrichtung der Ultrafiltrat-Pumpe umgekehrt.

Der Erfindung liegt die Aufgabe zugrunde, den Betrieb einer Blutbehandlungsvorrichtung in einem push-pull-Modus zu vereinfachen.

Die Lösung dieser Aufgabe erfolgt erfindungsgemäß mit den Merkmalen des Anspruch 1. Die abhängigen Ansprüche betreffen vorteilhafte Ausführungsformen der Erfindung.

Die Vereinfachung des Aufbaus der erfindungsgemäßen Vorrichtung zur extrakorporalen Blutbehandlung beruht darauf, die Ultrafiltrat-Pumpe der Ultrafiltrationseinrichtung in einem ersten und zweiten Betriebsmodus zu betreiben.

In dem ersten Betriebsmodus wird die Ultrafiltrat-Pumpe derart betrieben, dass der Druck auf der Blutseite der semipermeablen Membran größer als der Druck auf der Dialysierflüssigkeitsseite der semipermeablen Membran des Dialysators ist, so dass dem extrakorporalen Blutkreislauf während des ersten Betriebsmodus eine vorgegebene Menge an Flüssigkeit über die semipermeable Membran des Dialysators entzogen wird.

In dem zweiten Betriebsmodus wird die Ultrafiltrat-Pumpe derart betrieben, dass der Druck auf der Blutseite der semipermeablen Membran in aufeinanderfolgenden Intervallen wechselweise größer und kleiner als der Druck auf der Dialysierflüssigkeitsseite der semipermeablen Membran des Dialysators ist, so dass Flüssigkeit über die semipermeable Membran des Dialysators dem extrakorporalen Blutkreislauf laufend entzogen und zugeführt wird (push-pull-Modus).

Folglich sind bei der erfindungs gemäßen Vorrichtung zusätzliche Komponenten, insbesondere eine separate push-pull Pumpe, für den Betrieb im push-Pull-Modus nicht erforderlich. Daraus ergeben sich sowohl geringere Abmessungen für die Dialysemaschine als auch ein geringeres Gewicht. Mit dem Betrieb im push-pull-Modus kann die Standzeit des Dialysators erhöht werden, da sich Ablagerungen von der semipermeablen Membran des Dialysators lösen. Darüber hinaus kann für bestimmte Stoffe die Clearance der Dialyse erhöht werden.

Ein weiterer Vorteil liegt darin, dass die Änderung des Volumens (push-pull Volumen) nicht mit der Förderarte einer separaten push-pull Pumpe verknüpft ist, sondern mit der Förderrate der Ultrafiltratpumpe, die in die Bilanzierung von frischer und gebrauchter Dialysierflüssigkeit eingebunden ist.

Die Auswahl des jeweiligen Betriebsmodus kann bei der erfindungsgemäßen Vorrichtung mit einer Eingabeeinheit erfolgen, die auch Teil einer Menüführung sein kann.

Die Ultrafiltrat-Pumpe ist eine Verdrängerpumpe, die eine Pumpenkammer aufweist, in der ein beweglicher Verdrängerkörper angeordnet ist. Die Verdrängerpumpe ist vorzugsweise eine Kolbenpumpe oder eine Membranpumpe, mit der ein genau bestimmbares Volumen gefördert werden kann.

In dem ersten Betriebsmodus steht die Pumpenkammer der Verdrängerpumpe in einer vorausgehenden Saugphase mit dem Dialysierflüssigkeitssystem und in einer nachfolgenden Druckphase mit einem Ablauf in Fluidverbindung. In dem zweiten Betriebsmodus steht die Pumpenkammer der Verdrängerpumpe sowohl in der Saugphase als auch in der Druckphase mit dem Dialysierflüssigkeitssystem in Fluidverbindung, während die Fluidverbindung von der Pumpenkammer zu dem Ablauf unterbrochen ist.

Eine weitere bevorzugte Ausführungsform sieht vor, dass das Dialysierflüssigkeitssystem eine Bilanziereinrichtung umfasst, die eine oder mehrere Bilanzkammern aufweisen kann. Der Bilanziereinrichtung wird frische Dialysierflüssigkeit über eine erste Zuflussleitung aus einer Dialysierflüssigkeitsquelle zugeführt. Verbrauchte Dialysierflüssigkeit wird aus der Bilanziereinrichtung über eine Abflussleitung in einen Ablauf abgeführt. Von der Bilanziereinrichtung geht eine zweite Abflussleitung ab, die zu der Dialysierflüssigkeitskammer des Dialysators führt, und von der Dialysierflüssigkeitskammer führt eine zweite Zuflussleitung zu der Bilanziereinrichtung. Die Verdrängerpumpe ist bei dieser Ausführungsform an einer Ultrafiltrat-Leitung angeschlossen, die von der zweiten Zuflussleitung abzweigt und zu dem Ablauf führt.

Die Umschaltung zwischen dem ersten und zweiten Betriebsmodus erfolgt vorzugsweise mittels Absperrorganen, die in der Ultrafiltrat-Leitung stromauf bzw. stromab der Verdrängerpumpe angeordnet sind. Die Steuereinheit zum Ansteuern der Verdrängerpumpe und der Absperrorgane kann Bestandteil der zentralen Steuereinheit der Blutbehandlungsvorrichtung sein.

Eine besonders bevorzugte Ausführungsform sieht ein Dialysierflüssigkeitssystem vor, das einen die Dialysierflüssigkeitskammer des Dialysators einschließenden Dialysierflüssigkeitskreislauf aufweist, in dem die Dialysierflüssigkeit mehrfach durch die Dialysierflüssigkeitskammer gefördert wird, während dem Dialysierflüssigkeitssystem bilanziert frische und gebrauchte Dialysierflüssigkeit zugeführt wird. Bei dieser Ausführungsform wird die Blutbehandlungsvorrichtung vorteilhafterweise in dem zweiten Betriebsmodus betrieben, wenn die Dialysierflüssigkeit in dem Dialysierflüssigkeitskreislauf rezirkuliert.

Im Folgenden werden Ausführungsbeispiele der Erfindung unter Bezugnahme auf die Zeichnungen näher erläutert.

Es zeigen:
- Fig. 1: ein Ausführungsbeispiel der erfindungsgemäßen Blutbehandlungsvorrichtung in stark vereinfachter schematischer Darstellung, wobei der erste Arbeitstakt eines Arbeitszyklus dargestellt ist,
- Fig. 2: die Blutbehandlungsvorrichtung von Fig. 2, wobei der zweite Arbeitstakt des Arbeitszyklus dargestellt ist und
- Fig. 3: eine alternative Ausführungsform der Verdrängerpumpe der Ultrafiltrationseinrichtung.

Die Blutbehandlungsvorrichtung weist einen Dialysator 1 auf, der durch eine nicht dargestellte semipermeable Membran in eine nicht dargestellte Blutkammer und Dialysierflüssigkeitskammer unterteilt ist. Von dem Patienten führt eine Blutzuführleitung 2, in die eine Blutpumpe 3 geschaltet ist, zu einem Einlass 1A der Blutkammer des Dialysators 1, während von einem Auslass 1B der Blutkammer des Dialysators 1 eine Blutabführleitung 4 abgeht, die zu dem Patienten führt. Während der Blutbehandlung strömt das Blut des Patienten in dem extrakorporalen Blutkreislauf I durch die Blutkammer des Dialysators 1.

Zum Bilanzieren von frischer gegen verbrauchte Dialysierflüssigkeit ist eine Bilanziereinrichtung 5 vorgesehen, die bei dem vorliegenden Ausführungsbeispiel nur über eine Bilanzkammer 6 verfügt. Die Bilanzkammer 6 weist an der Unterseite einen ersten Einlass 6A und an der Oberseite einen ersten Auslass 6B sowie an der Unterseite einen zweiten Einlass 6C und an der Oberseite einen zweiten Auslass 6D auf.

Die frische Dialysierflüssigkeit wird in einer Dialysierflüssigkeitsquelle 7 bereitgestellt. Von der Dialysierflüssigkeitsquelle 7 geht eine erste Zuflussleitung 8 ab, die zu dem ersten Einlass 6A der Bilanzkammer 6 führt. Von dem ersten Auslass 6B der Bilanzkammer 6 geht eine erste Abflussleitung 9 ab, die zu einem Ablauf 32 führt. In die erste Zuflussleitung 8 ist eine Dialysierflüssigkeitspumpe 10, insbesondere eine okkludierende Pumpe, geschaltet, die frische Dialysierflüssigkeit aus der Dialysierflüssigkeitsquelle 7 in die Bilanzkammer 6 fördert.

Von dem zweiten Auslass 6D der Bilanzkammer 6 geht eine zweite Abflussleitung 11 ab, die zu dem Einlass 1C der Dialysierflüssigkeitskammer des Dialysators 1 führt. Von dem Auslass 1D der Dialysierflüssigkeitskammer geht eine zweite Zuflussleitung 12 ab, die zu dem zweiten Einlass 6C der Bilanzkammer 6 führt.

Bei den Zu- und Abflussleitungen 8, 9, 11, 12 handelt es sich um Schlauchleitungen, die auch als Kanäle in einer austauschbaren Einheit ausgebildet sein können. Die zweite Abflussleitung 11 weist in Strömungsrichtung einen ersten Abschnitt 11A und einen zweiten Abschnitt 11B auf, während die zweite Zuflussleitung 12 in Strömungsrichtung einen ersten Abschnitt 12A und einen zweiten Abschnitt 12B aufweist.

Die zweite Abflussleitung 11 und die zweite Zuflussleitung 12 sind über eine Bypass-Leitung 13 verbunden, die mit dem einen Ende an dem Verbindungspunkt 11C zwischen dem ersten Abschnitt 11A und dem zweiten Abschnitt 11B der zweiten Abflussleitung 11 und mit dem anderen Ende an dem Verbindungspunkt 12C zwischen dem ersten Abschnitt 12A und dem zweiten Abschnitt 12B der zweiten Zuflussleitung 12 angeschlossen ist.

Mit der Bypass-Leitung 13 wird ein Flüssigkeitskreislauf II geschaffen, der die Dialysierflüssigkeitskammer des Dialysators 1 einschließt. Der Flüssigkeitskreislauf II umfasst die Bypass-Leitung 13, den zweiten Abschnitt 11B der zweiten Abflussleitung 11, die Dialysierflüssigkeitskammer des Dialysators 1 und den ersten Abschnitt 12A der zweiten Zuflussleitung 12.

In den ersten Abschnitt 11A der zweiten Abflussleitung 11 ist eine zweite Pumpe 14 und in den zweiten Abschnitt 11B der zweiten Abflussleitung 11 eine dritte Pumpe 15 geschaltet.

Die Blutbehandlungsvorrichtung verfügt über eine Ultrafiltrationseinrichtung 16, die eine Ultrafiltrat-Pumpe 17 aufweist, mit der dem extrakorporalen Blutkreislauf I Flüssigkeit (Ultrafiltrat) entzogen werden kann.

Die Ultrafiltrat-Pumpe 17 ist an eine Ultrafiltrat-Leitung 18 angeschlossen, die von dem ersten Abschnitt 12A der zweiten Zuflussleitung 12 abgeht. Die Ultrafiltrat-Leitung 18 führt zu einem Ablauf 23, der beispielsweise ein Kanister sein kann.

Die Ultrafiltrat-Pumpe 17 ist bei dem vorliegenden Ausführungsbeispiel eine Kolbenpumpe, die eine Pumpenkammer aufweist, in der ein Kolben 24 längsverschiebbar angeordnet ist. Die Pumpenkammer 25 weist einen Ein-/Auslass 26 auf, der über eine Anschlussleitung 27 an die Ultrafiltrat-Leitung 18 angeschlossen ist, so dass die Pumpenkammer mit der Ultrafiltrat-Leitung in Fluidverbindung steht.

Die Pumpen 3, 10, 14, 15, 17 sind über Steuerleitungen 3', 10', 14', 15', 17' mit einer Steuereinheit 40 verbunden. Die Steuereinheit 40 ist bei dem vorliegenden Beispiel Bestandteil der zentralen Steuereinheit der Dialysevorrichtung.

In die erste Zuflussleitung 8 ist zwischen der ersten Pumpe 10 und der Bilanzkammer 6 ein erstes Absperrorgan 19 geschaltet, während in die erste Abflussleitung 9 ein zweites Absperrorgan 20 geschaltet ist. In die zweite Abflussleitung 11 ist zwischen der Bilanzkammer 6 und der zweiten Pumpe 14 ein drittes Absperrorgan 21 geschaltet, während in die zweite Zuflussleitung 12 zwischen dem Verbindungspunkt 12C und der Bilanzierkammer 6 ein viertes Absperrorgan 22 geschaltet ist.

In den Abschnitt der Ultrafiltrat-Leitung 18 stromauf des Anschlusspunktes der Anschlussleitung 27 an die Ultrafiltrat-Leitung ist ein fünftes Absperrorgan 28 und in den Abschnitt der Ultrafiltrat-Leitung stromab des Anschlusspunktes ist ein sechstes Absperrorgan 29 geschaltet.

Die Absperrorgane 19, 20, 21, 22, 28, 29 sind elektromagnetisch betätigbare Schlauchklemmen, die über Steuerleitungen 19', 20', 21', 22', 28', 29' mit der zentralen Steuereinheit 40 verbunden sind. Bei einer Ausführungsform mit einer austauschbaren Einheit, in der die Zu- und Abflussleitungen als Kanäle ausgebildet sind, können die Absperrorgane Ventile sein.

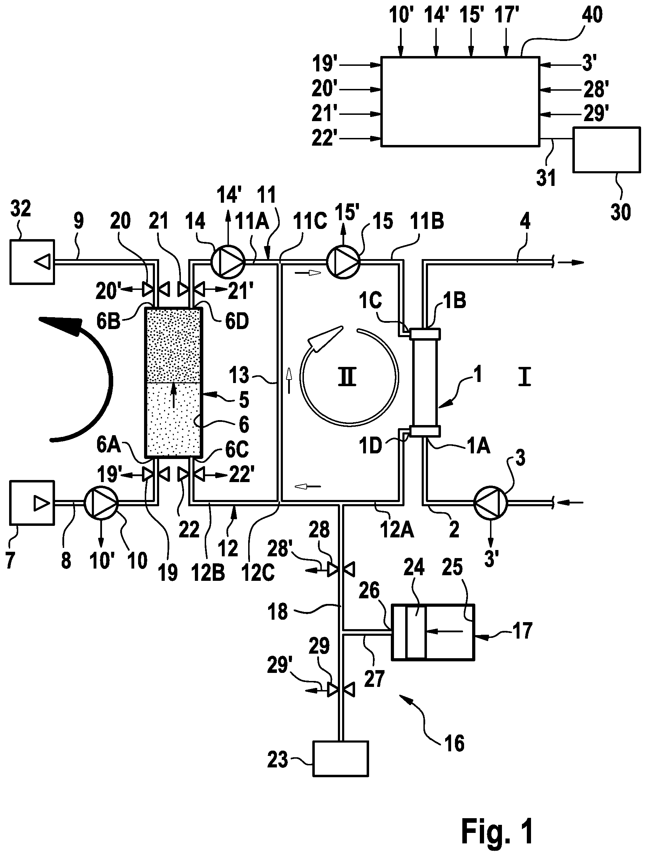

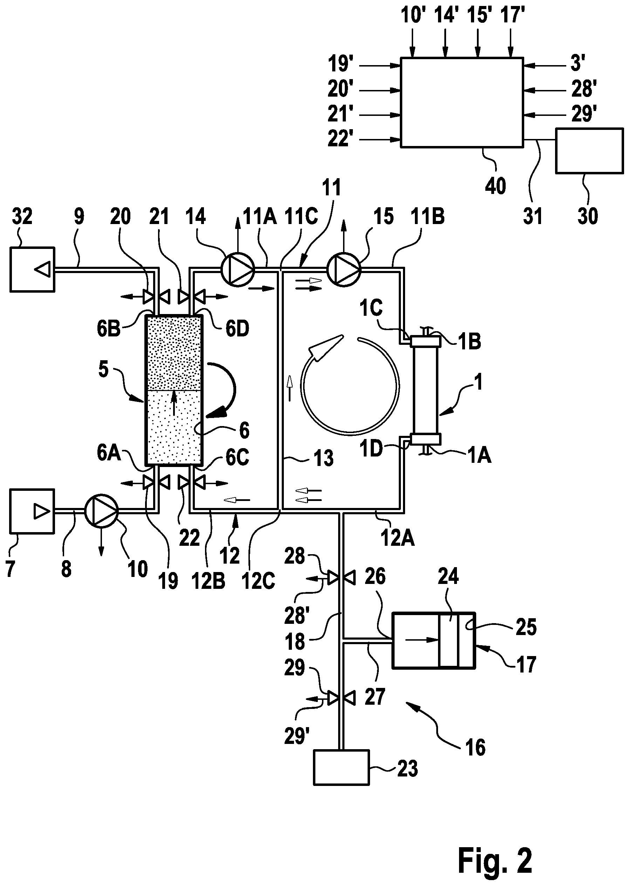

Für den Betrieb der Blutbehandlungsvorrichtung steuert die Steuereinheit 40 die Pumpen 10, 14, 15 und die Absperrorgane 19, 20, 21, 22 wie folgt an. Die Dialysevorrichtung wird in aufeinanderfolgenden Zyklen betrieben, die jeweils zwei Arbeitstakte umfassen. Fig. 1 zeigt den ersten Arbeitstakt und Fig. 2 den zweiten Arbeitstakt eines Arbeitszyklus.

Der erste Arbeitstakt umfasst das Befüllen der Bilanzierkammer 6, während Dialysierflüssigkeit durch den Dialysator 1 strömt. Die zentrale Steuereinheit 40 öffnet das erste und zweite Absperrorgan 19, 20 und schließt das dritte und vierte Absperrorgan 21, 22. Dabei setzt die Steuereinheit 40 die erste Pumpe 10 und die dritte Pumpe 15 in Betrieb. Die zweite Pumpe 14 steht still. Da die zweite Pumpe 14 eine okkludierende Pumpe ist, kann das dritte Absperrorgan 21 auch offen sein.

Die erste Pumpe 10 fördert frische Dialysierflüssigkeit aus der Dialysierflüssigkeitsquelle 7 in die Bilanzierkammer 6, die in dem zweiten Arbeitstakt des vorausgehenden Arbeitszyklus mit verbrauchter Dialysierflüssigkeit befüllt worden ist. Während sich die Bilanzkammer 6 mit frischer Dialysierflüssigkeit füllt, wird die verbrauchte Dialysierflüssigkeit über die erste Abflussleitung 9 in den Abfluss 32 verworfen. Die erste Pumpe 10 läuft solange, bis in der Bilanzkammer 6 verbrauchte Dialysierflüssigkeit vollständig gegen frische Dialysieflüssigkeit ausgetauscht ist. Während des Befüllens der Bilanzkammer 6 mit frischer Dialysierflüssigkeit ist die Flüssigkeitsströmung durch den Dialysator 1 nicht unterbrochen. Die dritte Pumpe 15 fördert die Dialysierflüssigkeit in dem Flüssigkeitskreislauf II, der den zweiten Abschnitt 11B der zweiten Abflussleitung 11, den Dialysator 1, den ersten Abschnitt 12A der zweiten Zuflussleitung 12 und die Bypass-Leitung 13 einschließt (Fig. 1A).

An den ersten Arbeitstakt (Fig. 1) schließt sich der zweite Arbeitstakt (Fig. 2) an. In dem zweiten Arbeitstakt schließt die zentrale Steuereinheit 40 das erste und zweite Absperrorgan 19, 20 und öffnet das dritte und vierte Absperrorgan 21, 22. Weiterhin hält die Steuereinheit 40 die erste Pumpe 10 an und setzt die zweite Pumpe 14 in Betrieb. Folglich laufen die zweite und dritte Pumpe 14, 15. Die Steuereinheit 40 gibt für die zweite Pumpe 14 eine kleinere Förderrate vor als für die dritte Pumpe 15. Folglich strömt in dem Flüssigkeitskreislauf II Dialysierflüssigkeit mit einer Flussrate, die der Differenz von den Förderraten der dritten und zweiten Pumpe 15, 14 entspricht. Diese Förderrate QD_{fast} kann relativ hoch sein.

Während Dialysierflüssigkeit in dem Flüssigkeitskreislauf II durch den Dialysator 1 zirkuliert, wird dem Flüssigkeitskreislauf II ständig frische Dialysierflüssigkeit zugeführt und verbrauchte Dialysierflüssigkeit dem Flüssigkeitskreislauf entzogen. Die frische Dialysierflüssigkeit wird mit der von der zweiten Pumpe 14 vorgegebenen Förderrate über den ersten Abschnitt 11A der zweiten Abflussleitung 11, die an den zweiten Auslass 6D der Bilanzierkammer 6 angeschlossen ist, dem Flüssigkeitskreislauf II zugeführt.

In Abhängigkeit von den Förderraten der zweiten und dritten Pumpe 14, 15 kann frische Dialysierflüssigkeit kontinuierlich in einem relativ kurzen oder einem relativ langen Zeitraum zugeführt und das gewünschte Verhältnis zwischen frischer und verbrauchter Dialysierflüssigkeit in dem Flüssigkeitskreislauf II eingestellt werden.

An den zweiten Arbeitstakt (Fig. 2) des Arbeitszyklus schließt sich dann wieder der erste Arbeitstakt (Fig. 1) eines nachfolgenden Zyklus an.

Weitere Ausführungsformen der Blutbehandlungsvorrichtung sind in der WO 2011/157396 beschrieben.

Die Blutbehandlungsvorrichtung weist eine Eingabeeinheit 30 auf, die mit der Steuereinheit 40 über eine Datenleitung 31 verbunden ist. Mit der Eingabeeinheit 30 kann ein erster oder zweiter Betriebsmodus ausgewählt werden, während die Blutbehandlungsvorrichtung wie oben beschrieben betrieben wird.

Für den ersten Betriebsmodus startet die Steuereinheit 40 die Kolbenpumpe 17. In der Saugphase der Pumpe 17 öffnet die Steuereinheit 40 das fünfte Absperrorgan 28 und schließt das sechste Absperrorgan 29 und in der Druckphase schließt die Steuereinheit 40 das fünfte Absperrorgan und öffnet das sechste Absperrorgan, so dass Ultrafiltrat dem extrakorporalen Blutkreislauf I während der Saugphase entzogen und während der Druckphase das Ultrafiltrat in den Ablauf abgeführt wird.

Für den zweiten Betriebsmodus startet die Steuereinheit 40 die Kolbenpumpe 17 und öffnet das fünfte Absperrorgan 29 und schließt das sechste Absperrorgan 30. Die Bewegung des Kolbens 24 der Kolbenpumpe 17 führt dazu, dass ein oszillierender Transmembrandruck erzeugt wird. Dabei wird ein dem Volumen der Pumpenkammer 25 entsprechendes Volumen an Flüssigkeit dem extrakorporalen Blutkreislauf I über die semipermeable Membran des Dialysators 1 wechselweise entzogen bzw. zugeführt, wodurch Ablagerungen an der semipermeablen Membran gelöst werden. Darüber hinaus wird die Clearance der Dialysebehandlung für bestimmte Stoffe erhöht.

Die Blutbehandlungsvorrichtung wird vorzugsweise in dem zweiten Betriebs-Modus (push-pull-Modus) betrieben, während die Dialysierflüssigkeit in dem Dialyiserflüssigkeitskreislauf II zirkuliert.

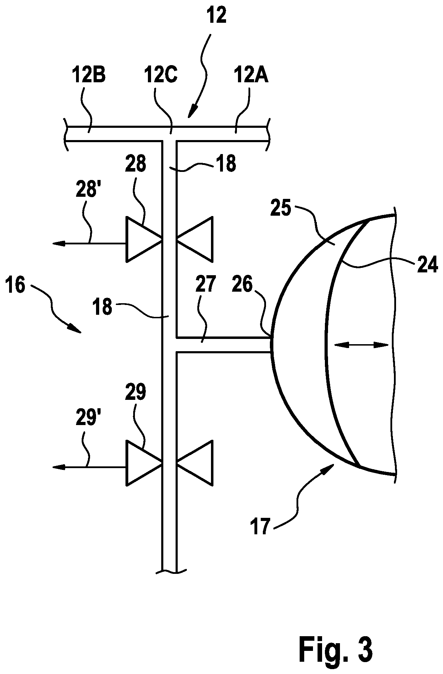

Fig. 3 zeigt eine alternative Ausführungsform der Verdrängerpumpe 17, wobei die einander entsprechenden Teile mit den gleichen Bezugszeichen versehen sind. Die Verdrängerpumpe 17 von Fig. 3 ist eine Membranpumpe, die eine Pumpenkammer 25 aufweist, in der als Verdrängerkörper eine flexible Membran 24 angeordnet ist. Die flexible Membran wird mit einer nicht dargestellten Druckmittelquelle mit einem vorgegebenen Druck beaufschlagt, so dass Flüssigkeit in der Pumpenkammer 25 verdrängt oder angesaugt wird. Dadurch ist es möglich, in dem Dialysierflüssigkeitssystem kontrolliert Druckpulse zu erzeugen, so dass Flüssigkeit wechselweise dem extrakorporalen Blutkreislauf I über die Membran des Dialysators entzogen oder zugeführt wird. Ansonsten unterscheidet sich die Membranpumpe in der Funktion nicht von der Kolbenpumpe.

## Patentansprüche

1. Vorrichtung zur extrakorporalen Blutbehandlung mit
einem Dialysator (1), der durch eine semipermeable Membran in eine Blutkammer und eine Dialysierflüssigkeitskammer getrennt ist,
einem die Blutkammer des Dialysators (1) einschließenden extrakorporalen Blutkreislauf (I),
einem die Dialysierflüssigkeitskammer des Dialysators (2) einschließenden Dialysierflüssigkeitssystem,
wobei das Dialysierflüssigkeitssystem eine Ultrafiltrationseinrichtung (16) umfasst, die eine Ultrafiltrat-Pumpe (17) aufweist, und eine Einrichtung zum Betreiben der Blutbehandlungsvorrichtung in einem ersten und zweiten Betriebsmodus vorgesehen ist, die derart ausgebildet ist, dass
in dem ersten Betriebsmodus die Ultrafiltrat-Pumpe (17) derart betrieben wird, dass der Druck auf der Blutseite der semipermeablen Membran größer als der Druck auf der Dialysierflüssigkeitsseite der semipermeablen Membran des Dialysators (1) ist, so dass dem extrakorporalen Blutkreislauf (I) während des ersten Betriebsmodus eine vorgegebene Menge an Flüssigkeit über die semipermeable Membran des Dialysators entzogen wird, und
in dem zweiten Betriebsmodus die Ultrafiltrat-Pumpe (17) derart betrieben wird, dass der Druck auf der Blutseite der semipermeablen Membran in aufeinanderfolgenden Intervallen wechselweise größer und kleiner als der Druck auf der Dialysierflüssigkeitsseite der semipermeablen Membran des Dialysators (1) ist, so dass Flüssigkeit über die semipermeable Membran des Dialysators dem extrakorporalen Blutkreislauf (I) laufend entzogen bzw. zugeführt wird, **dadurch gekennzeichnet, dass**
die Ultrafiltrat-Pumpe (17) eine Verdrängerpumpe ist, die eine Pumpenkammer (25) aufweist, in der ein beweglicher Verdrängerkörper (24) angeordnet ist, wobei die Einrichtung zum Betreiben der Blutbehandlungsvorrichtung in dem ersten und zweiten Betriebsmodus derart ausgebildet ist, dass
in dem ersten Betriebsmodus in einer vorausgehenden Saugphase der Verdrängerpumpe (17) die Pumpenkammer (25) der Verdrängerpumpe mit dem Dialysierflüssigkeitssystem in Fluidverbindung steht, und in einer nachfolgenden Druckphase die Pumpenkammer (25) mit einem Ablauf (23) in Fluidverbindung steht, und
in dem zweiten Betriebsmodus die Pumpenkammer (25) der Verdrängerpumpe (17) in der Saugphase und der Druckphase mit dem Dialysierflüssigkeitssystem in Fluidverbindung steht und die Fluidverbindung von der Pumpenkammer zu dem Ablauf (23) unterbrochen ist.

2. Vorrichtung nach Anspruch 1, **dadurch gekennzeichnet, dass** die Einrichtung zum Betreiben der Blutbehandlungsvorrichtung in dem ersten und zweiten Betriebsmodus eine Eingabeeinheit (30) zur Auswahl des ersten oder zweiten Betriebsmodus aufweist.

3. Vorrichtung nach Anspruch 1 oder 2, **dadurch gekennzeichnet, dass** die Verdrängerpumpe (17) eine Kolbenpumpe oder eine Membranpumpe ist.

4. Vorrichtung nach einem der Ansprüche 1 bis 3, **dadurch gekennzeichnet, dass** das Dialysierflüssigkeitssystem umfasst:
eine Bilanziereinrichtung (5),
eine zu der Bilanziereinrichtung (5) führende erste Zuflussleitung (8) zum Zuführen von Dialysierflüssigkeit aus einer Dialysierflüssigkeitsquelle (7) in die Bilanziereinrichtung und einer von der Bilanziereinrichtung abgehenden ersten Abflussleitung (9) zum Abführen von Dialysierflüssigkeit aus der Bilanziereinrichtung in einen Ablauf (32),
eine von der Bilanziereinrichtung (5) abgehende zweite Abflussleitung (11) zum Abführen von Dialysierflüssigkeit aus der Bilanziereinrichtung in die Dialysierflüssigkeitskammer des Dialysators (1) und einer zu der Bilanziereinrichtung führenden zweiten Zuflussleitung (12) zum Zuführen von Dialysierflüssigkeit aus der Dialysierflüssigkeitskammer in die Bilanziereinrichtung,
eine von zweiten Zuflussleitung (12) abzweigende und zu dem Ablauf (23) führenden Ultrafiltrat-Leitung (18),
wobei die Verdrängerpumpe (17) an die Ultrafiltrat-Leitung (18) angeschlossen ist.

5. Vorrichtung nach Anspruch 4, **dadurch gekennzeichnet, dass** die Einrichtung zum Betreiben der Blutbehandlungsvorrichtung in dem ersten und zweiten Betriebsmodus ein erstes Absperrorgan (28) aufweist, das in dem von der zweiten Zuflussleitung (12) zu der Pumpenkammer (25) der Verdrängerpumpe (17) führenden Abschnitt der Ultrafiltrat-Leitung (18) angeordnet ist und ein zweites Absperrorgan (29) aufweist, das in dem von der Pumpenkammer (25) abgehenden und zu dem Ablauf (23) führenden Abschnitt der Ultrafiltrat-Leitung (18) angeordnet ist.

6. Vorrichtung nach Anspruch 4 oder 5, **dadurch gekennzeichnet, dass** das Dialysierflüssigkeitssystem eine die zweite Abflussleitung (11) mit der zweiten Zuflussleitung (12) verbindende Bypass-Leitung (13) aufweist.

7. Vorrichtung nach einem der Ansprüche 5 oder 6, **dadurch gekennzeichnet, dass** die Einrichtung zum Betreiben der Blutbehandlungsvorrichtung in dem ersten und zweiten Betriebsmodus eine Steuereinheit (40) aufweist, die derart ausgebildet ist, dass die Verdrängerpumpe (17) und die Absperrorgane (28, 29) für den Betrieb der Blutbehandlungsvorrichtung in dem ersten oder zweiten Betriebsmodus angesteuert werden.

8. Vorrichtung nach Anspruch 7, **dadurch gekennzeichnet, dass** die Steuereinheit (40) derart ausgebildet ist, dass in dem ersten Betriebsmodus in der Saugphase der Verdrängerpumpe (17) das erste Absperrorgan (28) geöffnet und das zweite Absperrorgan (29) geschlossen ist, und in der Druckphase der Verdrängerpumpe das erste Absperrorgan (28) geschlossen und das zweite Absperrorgan (29) geöffnet ist

9. Vorrichtung nach Anspruch 7 oder 8, **dadurch gekennzeichnet, dass** die Steuereinheit (40) derart ausgebildet ist, dass in dem zweiten Betriebsmodus das erste Absperrorgan (28) geöffnet und das zweite Absperrorgan (29) geschlossen ist.

## Claims

1. Device for extracorporeal blood treatment, comprising
a dialyser (1), which is divided by a semipermeable membrane into a blood chamber and a dialysate chamber,
an extracorporeal blood circuit (I) including the blood chamber of the dialyser (1),
a dialysate system including the dialysate chamber of the dialyser (2),
the dialysate system comprising an ultrafiltration apparatus (16) which has an ultrafiltrate pump (17), and there is provided an apparatus for operating the blood treatment device in a first and second operating mode, which is formed in such a way that
the ultrafiltrate pump (17) is operated in the first operating mode in such a way that the pressure on the blood-side of the semipermeable membrane is higher than the pressure on the dialysate-side of the semipermeable membrane of the dialyser (1), so that during the first operating mode a predetermined amount of fluid is removed from the extracorporeal blood circuit (I) via the semipermeable membrane of the dialyser, and
in the second operating mode, the ultrafiltrate pump (17) is operated in such a way that the pressure on the blood-side of the semipermeable membrane is, at successive intervals, alternately higher and lower than the pressure on the dialysate-side of the semipermeable membrane of the dialyser (1), so that fluid is continuously removed from and supplied to the extracorporeal blood circuit (I) via the semipermeable membrane, respectively,
**characterised in that** the ultrafiltrate pump (17) is a positive displacement pump having a pumping chamber (25) in which a movable positive displacement element (24) is arranged, the apparatus for operating the blood treatment device in the first and second operating mode being formed in such a way that
in the first operating mode, the pumping chamber (25) of the positive displacement pump is in fluid communication with the dialysate system in a preceding suction phase of the positive displacement pump (17) and is in fluid communication with a drain (23) in a subsequent pressure phase of the pumping chamber (25), and
in the second operating mode, the pumping chamber (25) of the positive displacement pump (17) is in fluid communication with the dialysate system in both the suction phase and the pressure phase, and the fluid communication from the pumping chamber to the drain (23) is broken off.

2. Device according to claim 1, **characterised in that** the apparatus for operating the blood treatment device in the first and second operating mode has an input unit (30) for selecting either the first or second operating mode.

3. Device according to claim 1 or 2, **characterised in that** the positive displacement pump (17) is a reciprocating pump or a membrane pump.

4. Device according to any of claims 1 to 3, **characterised in that** the dialysate system comprises:
a balancing apparatus (5),
a first feed line (8) which leads to the balancing apparatus (5) and is intended for supplying dialysate from a dialysate source (7) into the balancing apparatus, and a first discharge line (9) which leads away from the balancing apparatus and is intended for carrying dialysate away from the balancing apparatus into a drain (32),
a second discharge line (11) which leads away from the balancing apparatus (5) and is intended to carry dialysate away from the balancing apparatus into the dialysate chamber of the dialyser (1), and a second feed line (12) which leads to the balancing apparatus and is intended to supply dialysate from the dialysate chamber into the balancing apparatus,
an ultrafiltrate line (18) which branches off from the second feed line (12) and leads to the drain (23),
the positive displacement pump (17) being joined to the ultrafiltrate line (18).

5. Device according to claim 4, **characterised in that** the apparatus for operating the blood treatment device in the first and second operating mode has a first obturating member (28), which is arranged in the portion of the ultrafiltrate line (18) which leads from the second feed line (12) to the pumping chamber (25) of the positive displacement pump (17), and a second obturating member (29), which is arranged in the portion of the ultrafiltrate line (18) which leads away from the pumping chamber (25) and to the drain (23).

6. Device according to either claim 4 or claim 5, **characterised in that** the dialysate system has a bypass line (13) which connects the second discharge line (11) to the second feed line (12).

7. Device according to either claim 5 or claim 6, **characterised in that** the apparatus for operating the blood treatment device in the first and second operating mode has a control unit (40) which is formed in such a way that the positive displacement pump (17) and the obturating members (28, 29) are controlled in the first and second operating mode for operation of the blood treatment device.

8. Device according to claim 7, **characterised in that** the control unit (40) is formed in such a way that in the first operating mode, in the suction phase of the positive displacement pump (17), the first obturating member (28) is open and the second obturating member (29) is shut and, in the pressure phase of the positive displacement pump, the first obturating member (28) is shut and the second obturating member (29) is open.

9. Device according to either claim 7 or claim 8, **characterised in that** the control unit (40) is formed in such a way that in the second operating mode the first obturating member (28) is open and the second obturating member (29) is shut.

## Revendications

1. Dispositif de traitement extracorporel du sang avec
un système de dialyse (1) qui est séparé par une membrane semi-perméable en une chambre de sang et une chambre de liquide de dialyse,
une circulation sanguine extracorporelle (I) incluant la chambre de sang du système de dialyse (1)
un système de liquide de dialyse incluant la chambre de liquide de dialyse du système de dialyse (2),
dans lequel le système de liquide de dialyse comprend une installation d'ultrafiltration (16) qui présente une pompe d'ultrafiltrat (17), et une installation pour faire fonctionner le dispositif de traitement du sang dans un premier et un deuxième mode de fonctionnement est prévue, qui est réalisée de telle sorte que dans le premier mode de fonctionnement, la pompe d'ultrafiltrat (17) fonctionne de telle sorte que la pression du côté sanguin de la membrane semi-perméable est supérieure à la pression sur le côté du liquide de dialyse de la membrane semi-perméable du système de dialyse (1), de sorte que, pendant le premier mode de fonctionnement, une quantité prédéterminée de liquide soit retirée de la circulation sanguine extracorporelle (I) par la membrane semi-perméable du système de dialyse, et
dans le deuxième mode de fonctionnement, la pompe d'ultrafiltrat (17) fonctionne de telle sorte que la pression du côté sanguin de la membrane semi-perméable dans des intervalles consécutifs est alternativement supérieure et inférieure à la pression sur le côté du liquide de dialyse de la membrane semi-perméable du système de dialyse (1) de sorte que du liquide soit retiré ou acheminé en continu par la membrane semi-perméable du système de dialyse de/à la circulation sanguine extracorporelle (I), **caractérisé en ce que**
la pompe d'ultrafiltrat (17) est une pompe volumétrique qui présente une chambre de pompe (25) dans laquelle un corps de refoulement mobile (24) est disposé, dans lequel l'installation pour le fonctionnement du dispositif de traitement du sang dans les premier et deuxième modes de fonctionnement est réalisée de telle sorte que dans le premier mode de fonctionnement dans une phase d'aspiration préalable de la pompe volumétrique (17), la chambre de pompe (25) de la pompe volumétrique est en communication fluidique avec le système de liquide de dialyse, et dans une phase de pression consécutive, la chambre de pompe (25) est en communication fluidique avec un écoulement (23), et
dans le deuxième mode de fonctionnement, la chambre de pompe (25) de la pompe volumétrique (17) est en communication fluidique, dans la phase d'aspiration et la phase de pression, avec le système de liquide de dialyse et la communication fluidique de la chambre de pompe à l'écoulement (23) est interrompue.

2. Dispositif selon la revendication 1, **caractérisé en ce que** l'installation pour le fonctionnement du dispositif de traitement du sang dans les premier et deuxième modes de fonctionnement présente une unité d'entrée (30) pour la sélection du premier ou deuxième mode de fonctionnement.

3. Dispositif selon la revendication 1 ou 2, **caractérisé en ce que** la pompe volumétrique (17) est une pompe à piston ou une pompe à membrane.

4. Dispositif selon l'une des revendications 1 à 3, **caractérisé en ce que** le système de liquide de dialyse comprend :
une installation d'équilibrage (5),
une première conduite d'afflux (8) conduisant à l'installation d'équilibrage (5) pour l'acheminement de liquide de dialyse issu d'une source de liquide de dialyse (7) dans l'installation d'équilibrage et d'une première conduite de drainage (9) partant de l'installation d'équilibrage pour l'évacuation du liquide de dialyse de l'installation d'équilibrage en un écoulement (32),
une deuxième conduite de drainage (11) partant de l'installation d'équilibrage (5) pour l'évacuation de liquide de dialyse de l'installation d'équilibrage dans la chambre de liquide de dialyse du système de dialyse (1) et d'une deuxième conduite d'afflux (12) conduisant à l'installation d'équilibrage pour l'acheminement de liquide de dialyse de la chambre de liquide de dialyse dans l'installation d'équilibrage,
une conduite d'ultrafiltrat (18) bifurquant de la deuxième conduite d'afflux (12) et conduisant à l'écoulement (23),
dans lequel la pompe volumétrique (17) est raccordée à la conduite d'ultrafiltrat (18).

5. Dispositif selon la revendication 4, **caractérisé en ce que** l'installation de fonctionnement du dispositif de traitement du sang présente, dans les premier et deuxième modes de fonctionnement, un premier organe de blocage (28) qui est disposé dans le segment de la conduite d'ultrafiltrat (18) conduisant de la deuxième conduite d'afflux (12) à la chambre de pompe (25) de la pompe volumétrique (17) et présente un deuxième organe de blocage (29) qui est disposé dans le segment de la conduite d'ultrafiltrat (18) partant de la chambre de pompe (25) et conduisant à l'écoulement (23).

6. Dispositif selon la revendication 4 ou 5, **caractérisé en ce que** le système de liquide de dialyse présente une conduite de dérivation (13) reliant la deuxième conduite de drainage (11) à la deuxième conduite d'afflux (12).

7. Dispositif selon l'une des revendications 5 ou 6, **caractérisé en ce que** l'installation de fonctionnement du dispositif de traitement du sang présente, dans les premier et deuxième modes de fonctionnement, une unité de commande (40) qui est réalisée de telle sorte que la pompe volumétrique (17) et les organes de blocage (28, 29) sont commandés pour le fonctionnement du dispositif de traitement du sang dans le premier ou deuxième mode de fonctionnement.

8. Dispositif selon la revendication 7, **caractérisé en ce que** l'unité de commande (40) est réalisée de telle sorte que dans le premier mode de fonctionnement dans la phase d'aspiration de la pompe volumétrique (17), le premier organe de blocage (28) est ouvert et le deuxième organe de blocage (29) est fermé, et dans la phase de pression de la pompe volumétrique, le premier organe de blocage (28) est fermé et le deuxième organe de blocage (29) est ouvert.

9. Dispositif selon la revendication 7 ou 8, **caractérisé en ce que** l'unité de commande (40) est réalisée de telle sorte que, dans le deuxième mode de fonctionnement, le premier organe de blocage (28) est ouvert et le deuxième organe de blocage (29) est fermé.
